# EUROPEAN PATENT SPECIFICATION

(11) **EP 3 629 771 B1**
(45) Date of publication and mention of the grant of the patent: **10.12.2025**
(21) Application number: 18739924.1
(22) Date of filing: 29.05.2018
(51) Int. Cl.: A23L 33/175, A61K 31/198, A61K 31/405, A61K 31/4172, A61K 9/46, A61K 9/00, A61K 9/10, A61K 9/20, A61K 9/16, A61K 9/48, A61K 9/50, A61P 17/02

(54) **AN ORAL NUTRITIONAL COMPOSITION FOR USE IN THE TREATMENT AND/OR PREVENTION OF PRESSURE ULCERS**
EINE ORALE ZUSAMMENSETZUNG FÜR DIE BEHANDLUNG UND/ODER PREVENTION VON DRUCKGESCHWÜREN
UNE COMPOSITION NUTRITIONNELLE ORALE DESTINÉE À LA PREVENTION ET/OU LE TRAITEMENT DES ESCARRES

(30) Priority: 02.06.2017 PL 42178017
(43) Date of publication of application: 08.04.2020
(73) Proprietor: Pellicarnos S.A., 00-351 Warszawa (PL)
(72) Inventor: GRIEB, Pawel, 02-201 Warszawa (PL); KRYCZKA, Tomasz Aleksander, 01-496 Warszawa (PL)
(74) Representative: Grzelak, Anna
(86) International application number: PCT/IB2018/053794
(87) International publication number: WO 2018/220518

(56) References cited:
- WO-A1-2016/012403
- US-A1- 2004 082 502
- US-A1- 2004 235 923
- US-A1- 2014 356 340
- US-A1- 2015 004 149
- US-A1- 2016 184 271
- DAWSON BERYL ET AL: "High Rate of Deficiency in the Amino Acids Tryptophan and Histidine in People with Wounds : Implication for Nutrient Targeting in Wound Management-A Pilot Study", ADVANCES IN SKIN AND WOUND CARE, vol. 22, no. 2, 1 February 2009 (2009-02-01), US, pages 79 - 82, XP093187039, ISSN: 1527-7941, DOI: 10.1097/01.ASW.0000345280.20779.17
- LEE H A ET AL: "Dialamine^(R) (essential amino acid powder) as a supplement to low protein diets in advanced chronic renal failure", CLINICAL NUTRITION, CHURCHILL LIVINGSTONE, LONDON, GB, vol. 6, no. 2, 1 May 1987 (1987-05-01), pages 111 - 116, XP022963272, ISSN: 0261-5614, [retrieved on 19870501], DOI: 10.1016/0261-5614(87)90030-6
- HIDEHARU YAMANAKA ET AL: "A multicenter, randomized, controlled study of the use of nutritional supplements containing collagen peptides to facilitate the healing of pressure ulcers", JOURNAL OF NUTRITION & INTERMEDIARY METABOLISM, vol. 8, 1 June 2017 (2017-06-01), pages 51 - 59, XP055502173, ISSN: 2352-3859, DOI: 10.1016/j.jnim.2017.05.001

## Description

### Technical field

The invention relates to an oral nutritional composition use in the treatment and/or prevention of pressure ulcers in patients with contraindications for a high-protein diet caused by renal failure by supplementing the patients with amino acids to be administered orally, preferably in form of modified release tablets, intended to compensate for a deficiency of amino acids in patients with pressure ulcers, in particular in patients with contraindications to take a high-protein diet; renal failure being the most common contraindication to take a high-protein diet.

### Background art

Pressure ulcers are localized damage to the skin and/or underlying tissues that form in immobilised patients, usually over a bony prominence, as a result of pressure or pressure in combination with friction and shear. They most commonly occur in the area of sacrum, coccyx, heels or hips. Other sites, such as elbows, knees, ankles or back of the cranium can also be affected. Pressure ulcers may develop in patients of all ages, but older patients are at particular risk. In this group of patients pressure ulcers often take the form of life-long non-healing chronic wounds. Such wounds are a particularly common problem in patients staying at long-term care centres, such as hospitals, care facilities or hospices.

Inadequate nutrient intake and symptoms of malnutrition, such as unwanted weight loss and low plasma albumin, are potentially modifiable risk factors for pressure ulcers. Therefore, international guidelines (National Pressure Ulcer Advisory Panel; 2009) recommend that multi-component dietary supplements containing *inter alia* proteins, amino acids and vitamins should be administered to patients with pressure ulcers. Known methods of supplementing the diet of patients with pressure ulcers and patients at risk of pressure ulcers involve administering high-protein nutritional compositions, often containing arginine (an amino acid) and zinc, antioxidants such as vitamins A, C and E, and other micronutrients (e.g. Cubitan^{®} manufactured by Nutricia, see also Cereda E. et al., Annals of Internal Medicine 2015;162:167-174).

However, high-protein diet is contraindicated to many patients with pressure ulcers or at risk of pressure ulcers, with chronic renal failure being the most common and most serious contraindication (Prevention and Treatment of Pressure Ulcers: Clinical Practice Guideline, Cambridge Media, 2014). Clinical observations reveal also that some patients with pressure ulcers do not show signs of malnutrition and the efficacy of the aforesaid high-protein dietary supplements remains limited in treatment of pressure ulcers in patients without any signs of malnutrition (van Anholt R.D. et al., Nutrition, 2010;26:867-872).

Scientific literature postulates that a composition of dietary supplements administered to patients with slow healing wounds, such as pressure ulcers, should be adjusted to a given deficiency to be remedied by these supplements (Wild T. et al., Nutrition, 2010; 26:862-866). In case of patients with pressure ulcers and contraindications for intake of high-protein preparations, it would be particularly favourable to administer only those amino acids the concentration of which is decreased, instead of a high-protein diet. The said contraindications are in most cases associated with chronic renal failure. However, in the prior art there are no known oral preparations intended for remedying deficiencies only of for those amino acids the concentration of which is decreased in patients with pressure ulcers.

In the publication Dawson Beryl et al. 'High rate deficiency in the amino acids tryptophan and histidine in people with wounds: implications for nutrient targeting in wound management - A pilot study', in Advanced in skin and wound care, vol.22, no.2, a low level of the essential amino acids tryptophan and histidine in people with wounds as an independent risk factor for the development of pressure ulcers in older adult patients and is associated with increased morbidity and death is discussed.

The publication 2004/0825002 A1concerns a method of treating a mammal to promote wound healing and administering an effective amounts of the essential amino acid composition in order to healing of wounds.

In publication "Dialamine^{®} (essential amino acid powder) as a supplement to low protein diets in advanced chronic renal failure" published in Clinical Nutrition, Churchill Livingstone, Londyn, GB, vol.6, no.2, (1987-05-01) there is disclosed that Dialamine R is an essential amino acid powder used as a supplement to low protein diets in patients with advanced chronic renal failure for whom dialysis is not possible for a variety of reasons. Thanks to supplementation well accepted by the patients, without side effects, their wellbeing and good physical health is maintained for long periods of time.

### Disclosure of the invention

This invention relates to a nutritional composition for use in the treatment and/or prevention of pressure ulcers in patients with contraindications for a high-protein diet caused by renal failure by supplementing the patients with amino acid, which composition is to be administered orally to patients with pressure ulcers or patients at risk of pressure ulcers, which contains the following amino acids: alanine, histidine, threonine and tryptophan as the only source of amino acids and as the active substance, as defined in the appended claims. Preferably in such a composition alanine and histidine may be replaced with an dipeptide L-carnosine, which in humans is absorbed into blood after oral administration and afterwards it is rapidly hydrolysed to its constituent amino acids, i.e. alanine and histidine.

Studies have unexpectedly shown that a nutritional composition containing these four amino acids is closely suited to the nature of deficiencies of amino acids reported in patients with pressure ulcers.

Oral supplementation of an amino acid deficiency with alanine, histidine, threonine and tryptophan, more preferably with L-carnosine (to replace alanine and histidine), threonine and tryptophan in order to treat and/or prevent pressure ulcers in patients who cannot receive recommended high-protein preparations unexpectedly turned out to be favourable. Additionally, a modified, sustained release composition form of the nutritional composition for use according to the invention to be used in patients for whom it is not advisable to take preparations that release peptides or amino acids in a rapid and uncontrolled manner in the gastrointestinal tract, which is why they are in a rapid and uncontrolled manner absorbed into the circulatory system, placing an additional burden on kidneys which are already failing, or other organs, the disrupted function thereof being a contraindication for using a high-protein diet.

The nutritional composition for use in the treatment and/or prevention of pressure ulcers in patients with contraindications for a high-protein diet caused by renal failure by supplementing the patients with amino acids is to be administered orally in order to remedy an amino acid deficiency, in order to treat and/or prevent pressure ulcers, which contains the following amino acids: alanine, histidine, threonine and tryptophan as the active ingredient.

The oral nutritional composition for use is used in order to treat and/or prevent pressure ulcers in patients with a contraindication for taking a high-protein diet; wherein the contraindication for taking a high-protein diet is renal failure.

The subject of the invention is an oral nutritional composition for use in the treatment and/or prevention of pressure ulcers in patients with contraindications for a high-protein diet caused by renal failure by supplementing the patients with amino acids, wherein the composition comprises as the only source of amino acids: alanine, histidine, threonine and tryptophan as the active substances, preferably wherein the active ingredient alanine and histidine are in the form of L-carnosine; and wherein said composition is in a form of a modified release composition with a slowed down, controlled and repeatable release rate of active substances in the gastrointestinal tract, and wherein the slowed down, controlled and repeatable release rate of the active substances is provided by at least one further additive being an agent slowing down the release of the active substance in the gastrointestinal tract, or a mixture or a combination of such additives.

In the preferred oral nutritional composition for use in the treatment and/or prevention of pressure ulcers in patients with contraindications for a high-protein diet caused by renal failure by supplementing the patients with amino acids the weight ratio of L-carnosine, threonine and tryptophan is 6:2:1.

In the preferred oral nutritional composition for use in the treatment and/or prevention of pressure ulcers in patients with contraindications for a high-protein diet caused by renal failure by supplementing the patients with amino acids the quantity of the active ingredients range from 50% to 80% of the total weight of the composition.

In the preferred oral nutritional composition for use in the treatment and/or prevention of pressure ulcers in patients with contraindications for a high-protein diet caused by renal failure by supplementing the patients with amino acids the agent slowing down the release of the active ingredient in the gastrointestinal tract is a coating substance and/or a substance forming a release retardant carrier.

In the preferred oral nutritional composition for use in the treatment and/or prevention of pressure ulcers in patients with contraindications for a high-protein diet caused by renal failure by supplementing the patients with amino acids the agent slowing down the release of the active ingredient in the gastrointestinal tract is a release retardant matrix system.

In the preferred oral nutritional composition for use in the treatment and/or prevention of pressure ulcers in patients with contraindications for a high-protein diet caused by renal failure by supplementing the patients with amino acids the composition has a form of a tablet, dragée, hard or soft capsule, soluble tablet, effervescent tablet, coated tablet, sugar-coated tablet, capsule, pellet, powder for reconstitution to a solution, oral suspension, microcapsule, two-phase tablet/capsule comprising a portion rapidly releasing and a portion slowly releasing the active ingredient, retard type tablet/capsule, chewable tablet or dragée.

In the preferred oral nutritional composition for use in the treatment and/or prevention of pressure ulcers in patients with contraindications for a high-protein diet caused by renal failure by supplementing the patients with amino acids the agent slowing down the release of the active ingredient is selected from a cellulose polymeric compound, ethyl cellulose, cellulose acetate, hydroxyethylcellulose, hydroxypropylmethylcellulose, methylcellulose, sodium carboxymethylcellulose, povidone, carmellose sodium, hypromellose, natural and modified gums, agar-agar, carbamate gum; alginates, alginic acid, polymers of acrylic acid, mucilages; molasses, mannose polysaccharides, galactose polysaccharides, chitosan, gelatin, starch, polyacrylates; waxes, lipids, mono-, di- and triglycerides of stearic and palmitic acid, stearic acid, polystyrene, polyvinyl chloride; calcium sulfate, anhydrous calcium di- and triphosphate, titanium oxide, colloidal silica; polyvinyl chloride, polyethylene, polystyrene, polyamides, silicone resins, Eudragit or mixtures thereof, preferably contains at least a combination of a cellulose polymer compound and povidone; preferably it contains at least a combination of hydroxypropylmethylcellulose and povidone.

The oral nutritional composition for use according to the invention is for oral use in order to supplement amino acid deficiencies in patients with pressure ulcers, in order to accelerate treatment of pressure ulcer-derived wounds or prevent pressure ulcers; it is for use in the treatment and/or prevention of pressure ulcers by administration to patients with contraindication for taking a high-protein diet caused by renal failure. The nutritional composition for use according to the invention comprises the following amino acids: alanine, histidine, threonine and tryptophan or L-carnosine, as a source of alanine and histidine, threonine and tryptophan. As observed, a deficiency of these 4 amino acids is associated with pressure ulcer occurrence, while supplementation thereof will result in reduced pressure ulcers and accelerated healing time, and will prevent new pressure ulcers from developing.

A diet rich in proteins and peptides is contraindicated to patients with numerous diseases and syndromes which often coincide with prolonged lying and the resulting pressure ulcers. The following should be listed in particular: renal failure, hepatic failure, cardiorespiratory failure, diabetes mellitus, cholelithiasis, metabolic disorders. The nutritional composition for use according to the invention, comprising only the amino acids necessary for healing pressure ulcers solves the aforesaid issue, does not place such a burden on the body as a high-protein diet, which is particularly the case with a modified sustained release composition which is recommended to patients with simultaneous pressure ulcers and renal failure.

Preferably, the oral nutritional composition for use according to the invention is a modified release composition which contains L-carnosine, threonine and tryptophan as active ingredient. A slowed release of L-carnosine in the gastrointestinal tract from a modified release oral nutritional composition, e.g. in the form of a tablet or capsule, increases exposure of human blood to this substance. Thereby, the time for competition between enzymatic hydrolysis in plasma and transport to organ tissues having oligopeptide-transporting proteins is extended, which consequently increases uptake of L-carnosine by the tissues and favourable effects thereof in humans after oral administration are enhanced.

The term "modified release" should be understood as an effect of a slowed down, controlled, sustained release of alanine, histidine, threonine and tryptophan, or more preferably L-carnosine, as a source of alanine and histidine, and threonine and tryptophan, from any oral pharmaceutical form that provides such an effect.

The effect of a modified, sustained release and consequently slower absorption may be achieved using various means known in the prior art, e.g. those selected from among solutions provided for in chapter 5, Oral drug delivery, p. 169-214, in a book entitled Drug Delivery Systems (ed. V.V. Randae and J.B. Cannon, CRC Press, 2011), wherein preferred is such a modified release nutritional composition or a capsule or tablet structure whereby the release rate of alanine, histidine, threonine and tryptophan, or more preferably L-carnosine, as a source of alanine and histidine, and threonine and tryptophan is maintained at a fairly steady rate and in time not shorter than 6 hours and not longer than 12 hours.

The effect of modified release of alanine, histidine, threonine and tryptophan, more preferably L-carnosine, as a source of alanine and histidine, and threonine and tryptophan may be achieved with the use of any agent for slowed, sustained release and thereby slower absorption of the active ingredient. For example, the release retarding agent in the composition for use of the invention will be the known systems involving coating methods, incorporating the active compounds or complexing thereof by forming a sparingly soluble complex of the active compounds with a macromolecular compound.

For the release retarding agent used in coating methods, additives such as coating substances may be used, e.g. methylcellulose, polyvidone. Release retarding agents that are formed by incorporating alanine, histidine, threonine and tryptophan, more preferably L-carnosine, as a source of alanine and histidine, and threonine and tryptophan, will involve a release retardant carrier (additive), e.g. a hydrophilic carrier (e.g. methylcellulose, carmellose sodium, hypromellose, alginates, acrylic acid polymers, vegetable mucilages), a lipophilic carrier (e.g. waxes, mono-, di- and triglycerides of stearic and palmitic acid, stearic acid), a carrier that is insoluble in the gastrointestinal tract (e.g. an inorganic compound such as calcium sulphate, anhydrous calcium di- and triphosphate, titanium oxide, colloidal silica; an organic compound like polyvinyl chloride, polyethylene, cellulose acetate, ethyl cellulose, polystyrene, polyamides, silicone resins, Eudragit RL, RS). The release retardant agent may also be a matrix system (multilayer tablets, coated tablets).

The examples of polymeric compounds used in a modified release hydrophilic matrix include cellulose derivatives of high viscosity, e.g.: hydroxyethylcellulose, hydroxypropylmethylcellulose, methylcellulose or sodium carboxymethylcellulose; as well as other compounds not being cellulose-derivatives: agar-agar, carbamate gum, alginic acid, alginates, molasses, mannose and galactose polysaccharides, chitosan, gelatin, natural and modified gums, modified starch or polyacrylates (Carbopol^{®} 934). Preferably, the polymeric compound providing slowed release of the active ingredient is hydroxypropylmethylcellulose, more preferably a mixture of two hydroxypropylmethylcelluloses of different viscosity. Most preferably, the polymeric compound providing slowed release of the active ingredient is a mixture of hydroxypropylmethylcellulose with a viscosity of approx. 4000 cP and hydroxypropylmethylcellulose with a viscosity of approx. 100 cP.

In the oral nutritional composition for use according to the invention, the percentage of a cellulose polymer may preferably be in a range from 10% to 40% of the total tablet weight and more preferably from 20% to 30% of the total tablet weight.

In the oral nutritional composition for use of the invention, in order to modify the release of alanine, histidine, threonine and tryptophan, more preferably L-carnosine, as a source of alanine and histidine, and threonine and tryptophan, a polymeric compound may be used, forming a hydrophobic matrix, alanine, histidine, threonine and tryptophan, or more preferably L-carnosine, as a source of alanine and histidine, and threonine and tryptophan being gradually passed therefrom to gastric juice by diffusion. The hydrophobic polymer compound used is preferably: wax, lipid, ethyl cellulose, cellulose acetate, polystyrene, polyvinyl chloride, etc.

In order to modify the release of alanine, histidine, threonine and tryptophan, more preferably L-carnosine, as a source of alanine and histidine, and threonine and tryptophan, the oral nutritional composition for use according to the invention may also include a combination of hydrophilic and hydrophobic compounds, e.g. the active ingredient may be covered by a mixture of ethyl- and methylcellulose. While dissolving in water, methylcellulose forms pores in the insoluble ethyl cellulose membrane, which enables gradual release of alanine, histidine, threonine and tryptophan, more preferably L-carnosine as a source of alanine and histidine, and threonine and tryptophan from within.

The oral nutritional compositions for use, allowing modified release of alanine, histidine, threonine and tryptophan, more preferably L-carnosine as a source of alanine and histidine, and threonine and tryptophan, may also utilize bioadhesive or mucoadhesive polymers, such as acrylic acid or chitosan.

In order to modify the release of alanine, histidine, threonine and tryptophan, more preferably L-carnosine as a source of alanine and histidine, and threonine and tryptophan, the oral nutritional composition for use according to the invention may also include buffering compounds, which weaken the interdependence between release of the active ingredient and pH changes and ensure a constant release rate of alanine, histidine, threonine and tryptophan, more preferably L-carnosine as a source of alanine and histidine, and threonine and tryptophan, along long sections of the digestive system. The examples of such buffering compounds may include salts of phosphoric acid, tartaric acid or citric acid.

Pharmaceutically acceptable additives, such as binding agents, preferably e.g. povidone (polyvinylpyrrolidone), and lubricants (e.g. magnesium stearate) may be used in the oral nutritional composition for use according to the invention.

As far as practical realization of the present invention is concerned, there are no particular limitations as to the content of alanine, histidine, threonine and tryptophan, more preferably L-carnosine as a source of alanine and histidine and threonine and tryptophan, in tablets or capsules, but preferably unit doses of alanine are in a range from 50 mg to 500 mg, more preferably from 100 mg to 400 mg, and more preferably from 200 mg to 300 mg; unit doses of histidine are in a range from 50 mg to 500 mg, more preferably from 100 mg to 400 mg, and more preferably from 200 mg to 300 mg, whereas unit doses of L-carnosine as a source of alanine and histidine, are in a range from 100 mg to 1000 mg, more preferably from 200 mg to 800 mg, and more preferably from 400 mg to 600 mg; preferable unit doses of threonine are in a range from 10 mg to 600 mg, more preferably from 50 mg to 400 mg, and more preferably from 100 mg to 200 mg; preferable unit doses of tryptophan are in a range from 10 mg to 400 mg, more preferably from 20 mg to 200 mg, and more preferably from 50 mg to 100 mg.

The modified-release oral nutritional composition for use with alanine, histidine, threonine and tryptophan, more preferably L-carnosine as a source of alanine and histidine, and threonine and tryptophan may be in a form of a tablet, dragée, hard or soft capsule, soluble tablet, effervescent tablet, coated tablet, sugar-coated tablet, capsule, pellet, powder for reconstitution in a solution, oral suspension, microcapsule, two-phase tablet/capsule comprising a portion rapidly releasing and a portion slowly releasing the active ingredient, retard tablet/capsule, chewable tablet or dragée, etc.

The examples below illustrate the invention, not limiting it in any manner.

### Examples

### Example 1

### Analysis of blood amino acid levels in patients with pressure ulcers

Blood samples were collected from patients with comparable and similarly advanced diseases (terminal states of various cancer types). Serum was isolated from these blood samples. Subsequently, in these samples, after derivatisation of the analytes with o-phthalaldehyde and mercaptoethanol 14 amino acids were assayed by high pressure chromatography (HPLC) with a fluorescence detector. The results obtained for patients with pressure ulcers were compared with the results obtained for patients without pressure ulcers. Unexpectedly, it turned out (Table 1) that the concentration of four amino acids included in the composition for use according to this invention, i.e. alanine, histidine, threonine and tryptophan, is lower in the serum of patients with pressure ulcers than in patients without pressure ulcers, while the concentrations of other amino acids do not differ between these groups of patients.

Since patients were classified into groups based only on presence or absence of pressure ulcers, this result indicates that patients with pressure ulcers have a specific deficiency of the four amino acids. This means that a nutritional composition for use according to this invention is suited for the specific nature of amino acid deficiency in patients with pressure ulcers compared to patients without pressure ulcers. The amino acids showing a marked deficiency in patients with pressure ulcers include both essential amino acids threonine and tryptophan, which are not synthesised in the human body and must be provided with food; histidine, a conditionally essential amino acid, the synthesis thereof possibly being insufficient in the human body; and alanine, an endogenous amino acid, which has been believed to be synthesised in sufficient quantities in human body.

**Table 1. Serum amino acid concentrations in patients without pressure ulcers (n=43) and patients with pressure ulcers (n=22); mean values +/- SE [µM].**

| **Amino acid** | **Without pressure ulcers** | **With pressure ulcers** | **p** |
|---|---|---|---|
| **alanine** | 195.59 +/- 11.95 | 144.88 +/- 12.59 | **0.016** |
| arginine | 40.75 +/- 2.38 | 33.31 +/- 2.65 | 0.057 |
| citrulline | 21.98 +/- 1.26 | 19.25 +/- 1.57 | 0.199 |
| phenylalanine | 46.04 +/- 2.24 | 49.48 +/- 3.08 | 0.372 |
| glutamine | 487.22 +/- 21.55 | 415.78 +/- 31.22 | 0.061 |
| **histidine** | 36.65 +/- 1.37 | 31.73 +/- 1.81 | **0.037** |
| isoleucine | 53.20 +/- 3.68 | 51.07 +/- 5.11 | 0.510 |
| aspartic acid | 3.57 +/- 0.18 | 3.16 +/- 0.15 | 0.324 |
| leucine | 83.34 +/- 5.30 | 89.51 +/- 8.86 | 0.884 |
| ornithine | 50.17 +/- 3.09 | 46.55 +/- 3.38 | 0.469 |
| serine | 71.19 +/- 3.04 | 59.87 +/- 3.64 | 0.060 |
| **threonine** | 81.43 +/- 4.91 | 57.34 +/- 4.18 | **0.008** |
| **tryptophan** | 25.28 +/- 1.78 | 18.95 +/- 1.43 | **0.022** |
| valine | 158.33 +/- 7.48 | 151.30 +/- 12.82 | 0.615 |

| | | | |
|---|---|---|---|
| p - probability that there is no difference between the groups, determined using the Student's t test for independent samples (provided that the distribution of data in both groups was normal) or the Mann-Whitney U test. | | | |

This observation indicates that deficiencies of these amino acids should be orally supplemented in patients with pressure ulcers and patients at risk of pressure ulcers. In order to counteract the deficiency of these four amino acids, it is not necessary to administer high-protein supplements to patients as it will be sufficient to administer a nutritional composition containing alanine, histidine, threonine and tryptophan, more preferably L-carnosine as a source of alanine and histidine, and threonine and tryptophan.

### Example 2

### Production of a tablet containing alanine, histidine, threonine and tryptophan for supplementation of amino acid deficiencies in patients with pressure ulcers

Equimolar portions of individual amino acids: 89.09 g of alanine, 155.15 g of histidine, 119.12 g of threonine and 204.23 g of tryptophan were thoroughly mixed dry, preparing thereby a homogeneous mixture. The obtained powder was supplemented with a granulating liquid, being a solution of 3.5 g of povidone (polyvinylpyrrolidone, binding agent) in150 mL of water, and subjected to a granulation cycle. The obtained suspension was ground wet and subsequently dried on a fluidized bed. The obtained granulate was sieved through a 0.16-0.25 mm sieve, mixed with 2.5 g of magnesium stearate (lubricant) and this mixture was formed into tablets using a laboratory tablet press with flat punches with a diameter of 13 mm, thereby obtaining tablets with nominal mass of 1000 mg and equimolar content of individual amino acids.

Thereby, the nutritional composition of alanine, histidine, threonine and tryptophan, in the form of a tablet with equimolar content of individual amino acids was obtained.

### Example 3

### Production of a modified-release tablet containing alanine, histidine, threonine and tryptophan for supplementation of amino acid deficiencies in patients with pressure ulcers

Equimolar portions of individual amino acids: 89.09 g of alanine, 155.15 g of histidine, 119.12 g of threonine, 204.23 g of tryptophan and 300.0 g of hypromellose (hydroxypropylmethylcellulose, Methocel K4M offered by Colorcon, a substance that prolongs release and thereby slows absorption) were thoroughly mixed dry, preparing thereby a homogeneous mixture. The obtained powder was supplemented with a granulating liquid, being a solution of 3.5 g of povidone (polyvinylpyrrolidone, binding agent) in 150 mL of water and subjected to a granulation cycle. The obtained suspension was ground wet and subsequently dried on a fluidized bed. The obtained granulate was sieved through a 0.16-0.25 mm sieve, mixed with 2.5 g of magnesium stearate (lubricant) and this mixture was formed into tablets using a laboratory tablet press with flat punches with a diameter of 13 mm, thereby obtaining tablets with nominal mass of 1000 mg and equimolar content of individual amino acids.

Thereby, the nutritional composition of alanine, histidine, threonine and tryptophan, , in the form of a modified-release tablet with equimolar content of individual amino acids was obtained.

### Example 4

### Production of a modified-release tablet containing L-carnosine, threonine and tryptophan for supplementation of amino acid deficiencies in patients with pressure ulcers

Portions of 500.0 g of L-carnosine, 167.0 g of threonine, 83.0 g of tryptophan and 300.0 g of hypromellose (hydroxypropylmethylcellulose, Methocel K4M offered by Colorcon, a substance that prolongs release and thereby slows absorption) were thoroughly mixed dry, preparing thereby a homogeneous mixture. The obtained powder was supplemented with a granulating liquid, being a solution of 3.5 g of povidone (polyvinylpyrrolidone, binding agent) in 150 mL of water and subjected to a granulation cycle. The suspension was ground wet and subsequently dried on a fluidized bed. The obtained granulate was sieved through a 0.16-0.25 mm sieve, mixed with 2.5 g of magnesium stearate (lubricant) and this mixture was formed into tablets using a laboratory tablet press with flat punches with a diameter of 13 mm, thereby obtaining tablets with nominal mass of 1056 mg and nominal content of 500 mg of L-carnosine, 167 mg of threonine and 83 mg of tryptophan.

Thereby the nutritional composition, containing L-carnosine as a source of alanine and histidine, and threonine and tryptophan, in the form of a modified-release tablet was obtained. Apart from threonine and tryptophan, the nutritional composition also comprises the L-carnosine dipeptide, which is absorbed in the small intestine if administered orally and rapidly hydrolysed to its constituent amino acids in plasma.

Administration of the nutritional composition, particularly in a modified-release formcontaining alanine and histidine, more preferably L-carnosine as a source of alanine and histidine, and threonine and tryptophan, produced according to example 2, 3, 4 makes it possible to supplement patients with pressure ulcers with alanine, histidine, threonine and tryptophan. Such supplementation turned out to be particularly favourable in patients with pressure ulcers and contraindications for a high-protein diet, as using such a preparation not only accelerates their healing of pressure ulcer-derived wounds or prevents formation of new ones but also no excess amino acid or peptide load occurs in the body as is the case when on a high-protein diet in order to supplement amino acids, a deficiency of which was observed in patients with pressure ulcers. It was noted that the nutritional composition, particularly in a modified-release form, has a favourable effect on the treatment of pressure ulcers and/or prevention of pressure ulcer-derived wounds in studied patients. Use of the nutritional composition, in a modified-release form, has a particularly preferred effect on the treatment of pressure ulcers and/or prevention of formation of pressure ulcer-derived wounds in patients with concomitant renal failure, hepatic failure, cardiorespiratory failure, diabetes mellitus, cholelithiasis, metabolic disorders, particularly preferable being the use of the modified release nutritional composition in patients with impaired renal function and pressure ulcers.

Therefore, the use of the modified-release nutritional composition, e.g. manufactured according to example 3, is even more preferable compared to supplementation with typical tablets or capsules, particularly preferable being the use of the modified-release nutritional composition with L-carnosine as a source of alanine and histidine, and threonine and tryptophan, produced e.g. according to example 4, because its administration results in a steadily extended supplementation with L-carnosine as a source of alanine and histidine, and threonine and tryptophan, compared to supplementation with typical tablets or capsules, which release substances in the gastrointestinal tract in an uncontrolled manner in a form of a single high dose. Furthermore, administration of L-carnosine additionally stimulates healing of wounds, including pressure ulcer-derived wounds.

Therefore, the modified-release nutritional composition is particularly preferably intended for the treatment and/or prevention of pressure ulcers in patients by supplementing them only with the deficient amino acids required for healing of wounds associated with pressure ulcers. Its administration turned out to be particularly favourable in patients with contraindications for a high-protein diet, resulting from impaired renal function.

### References:

van Anholt RD, Sobotka L, Meijer EP, Heyman H, Groen HW, et al., Specific nutritional support accelerates pressure ulcer healing and reduces wound care intensity in non-malnourished patients. Nutrition, 2010; 26: 867-872.;
Cereda E, Klersy E, Andreola M, Pisati R, Schols J, Caccialanza R, D'Andrea F, Cost-effectiveness of a disease-specific oral nutritional suport for pressure ulcer healing, Clinical Nutrition 2017; 36: 246-252;
Dawson B, Favaloro E, High rate of deficiency in the amino acids tryptophan and histidine in people with wounds: implication for nutrient targeting in wound management - a pilot study, Advances in Skin & Wound Care 2009; 22: 79-82;
European Pressure Ulcer Advisory Panel and National Pressure Ulcer Advisory Panel. Prevention and treatment of pressure ulcers: quick reference guide. Washington, DC: National Pressure Ulcer Advisory Panel; 2009;
Gardner ML, Illingworth KM, Kelleher J, Wood D, Intestinal absorption of the intact peptide carnosine in man, and comparison with intestinal permeability to lactulose. J Physiol 1991;439: 411-422;
Wild T, Rahbarnia A, Kellner M, Sobotka L, Eberlein T, Basics in nutrition and wound healing. Nutrition 2010; 26: 862-866.

## Claims

1. An oral nutritional composition for use in the treatment and/or prevention of pressure ulcers in patients with contraindications for a high-protein diet caused by renal failure by supplementing the patients with amino acids,
wherein the composition comprises
as the only source of amino acids: alanine, histidine, threonine and tryptophan as the active substances, preferably wherein the active ingredient alanine and histidine are in the form of L-carnosine; and
wherein said composition is in a form of a modified release composition with a slowed down, controlled and repeatable release rate of active substances in the gastrointestinal tract, and wherein the slowed down, controlled and repeatable release rate of the active substances is provided by at least one further additive being an agent slowing down the release of the active substance in the gastrointestinal tract, or a mixture or a combination of such additives.

2. The oral nutritional composition for use according to claim 1, wherein the weight ratio of L-carnosine, threonine and tryptophan is 6:2:1.

3. The oral nutritional composition for use according to claims 1-2, wherein the quantity of the active ingredients range from 50% to 80% of the total weight of the composition.

4. The oral nutritional composition for use according to claims 1-3, wherein the agent slowing down the release of the active ingredient in the gastrointestinal tract is a coating substance and/or a substance forming a release retardant carrier.

5. The oral nutritional composition for use according to claims 1-4, wherein the agent slowing down the release of the active ingredient in the gastrointestinal tract is a release retardant matrix system.

6. The oral nutritional composition for use according to claims 1-5, wherein it has a form of a tablet, dragée, hard or soft capsule, soluble tablet, effervescent tablet, coated tablet, sugar-coated tablet, capsule, pellet, powder for reconstitution to a solution, oral suspension, microcapsule, two-phase tablet/capsule comprising a portion rapidly releasing and a portion slowly releasing the active ingredient, retard type tablet/capsule, chewable tablet or dragée.

7. The oral nutritional composition for use according to claims 1-6, wherein the agent slowing down the release of the active ingredient is selected from a cellulose polymeric compound, ethyl cellulose, cellulose acetate, hydroxyethylcellulose, hydroxypropylmethylcellulose, methylcellulose, sodium carboxymethylcellulose, povidone, carmellose sodium, hypromellose, natural and modified gums, agar-agar, carbamate gum; alginates, alginic acid, polymers of acrylic acid, mucilages; molasses, mannose polysaccharides, galactose polysaccharides, chitosan, gelatin, starch, polyacrylates; waxes, lipids, mono-, di- and triglycerides of stearic and palmitic acid, stearic acid, polystyrene, polyvinyl chloride; calcium sulfate, anhydrous calcium di- and triphosphate, titanium oxide, colloidal silica; polyvinyl chloride, polyethylene, polystyrene, polyamides, silicone resins, Eudragit or mixtures thereof.

8. The oral nutritional composition for use according to claim 7, **characterised in that** it contains at least a combination of a cellulose polymer compound and povidone; preferably it contains at least a combination of hydroxypropylmethylcellulose and povidone.

## Patentansprüche

1. Orale Nahrungszusammensetzung zur Verwendung bei der Behandlung und/oder Vorbeugung von Druckgeschwüren bei Patienten mit Kontraindikationen für eine proteinreiche Ernährung aufgrund von Nierenversagen, indem den Patienten Aminosäuren zugeführt werden, wobei die Zusammensetzung als einzige Aminosäurequelle Alanin, Histidin, Threonin und Tryptophan als Wirkstoffe enthält, vorzugsweise wobei die Wirkstoffe Alanin und Histidin in Form von L-Carnosin vorliegen; und
wobei die Zusammensetzung in Form einer Zusammensetzung mit modifizierter Freisetzung vorliegt, mit einer verlangsamten, kontrollierten und wiederholbaren Freisetzungsrate der Wirkstoffe im Magen-Darm-Trakt, und wobei die verlangsamte, kontrollierte und wiederholbare Freisetzungsrate der Wirkstoffe durch mindestens einen weiteren Zusatzstoff bereitgestellt wird, der ein Mittel ist, das die Freisetzung des Wirkstoffs im Magen-Darm-Trakt verlangsamt, oder durch eine Mischung oder Kombination solcher Zusatzstoffe.

2. Orale Nahrungszusammensetzung zur Verwendung gemäß Anspruch 1, wobei das Gewichtsverhältnis von L-Carnosin, Threonin und Tryptophan 6:2:1 beträgt.

3. Orale Nahrungszusammensetzung zur Verwendung gemäß den Ansprüchen 1-2, wobei die Menge der Wirkstoffe zwischen 50 % und 80 % des Gesamtgewichts der Zusammensetzung liegt.

4. Orale Nahrungszusammensetzung zur Verwendung gemäß den Ansprüchen 1-3, wobei das Mittel, das die Freisetzung des Wirkstoffs im Magen-Darm-Trakt verlangsamt, eine Beschichtungssubstanz und/oder eine Substanz ist, die einen freisetzungsverzögernden Träger bildet.

5. Orale Nahrungszusammensetzung zur Verwendung gemäß den Ansprüchen 1-4, wobei das Mittel, das die Freisetzung des Wirkstoffs im Magen-Darm-Trakt verlangsamt, ein freisetzungsverzögerndes Matrixsystem ist.

6. Orale Die orale Nahrungszusammensetzung zur Verwendung gemäß den Ansprüchen 1-5, wobei sie die Form einer Tablette, Dragée, Hart- oder Weichkapsel, löslichen Tablette, Brausetablette, beschichteten Tablette, zuckerbeschichteten Tablette, Kapsel, Pellets, Pulver zur Herstellung einer Lösung, oralen Suspension, Mikrokapsel, Zweiphasentablette/Kapsel, die einen schnell freisetzenden Teil und einen langsam freisetzenden Teil des Wirkstoffs umfasst, Retardtablette/Kapsel, Kautablette oder Dragée hat.

7. Orale Nahrungszusammensetzung zur Verwendung gemäß den Ansprüchen 1-6, wobei das Mittel, das die Freisetzung des Wirkstoffs verlangsamt, aus Folgendem ausgewählt ist: Cellulosepolymerverbindung, Ethylcellulose, Celluloseacetat, Hydroxyethylcellulose, Hydroxypropylmethylcellulose, Methylcellulose, Natriumcarboxymethylcellulose, Povidon, Carmellose-Natrium, Hypromellose, natürlichen und modifizierten Gummis, Agar-Agar, Carbamate; Alginaten, Alginsäure, Polymeren von Acrylsäure, Schleimstoffen; Melasse, Mannose-Polysaccharide, Galactose-Polysaccharide, Chitosan, Gelatine, Stärke, Polyacrylate; Wachsen, Lipiden, Mono-, Di- und Triglyceriden von Stearin- und Palmitinsäure, Stearinsäure, Polystyrol, Polyvinylchlorid; Calciumsulfat, wasserfreiem Calciumdi- und - triphosphat, Titanoxid, kolloidalem Siliciumdioxid; Polyvinylchlorid, Polyethylen, Polystyrol, Polyamide, Silikonharze, Eudragit oder Mischungen davon.

8. Orale Nahrungszusammensetzung zur Verwendung gemäß Anspruch 7, **dadurch gekennzeichnet, dass** sie mindestens eine Kombination aus einer Cellulosepolymerverbindung und Povidon enthält; vorzugsweise enthält sie mindestens eine Kombination aus Hydroxypropylmethylcellulose und Povidon.

## Revendications

1. Composition orale nutritionnelle à utiliser dans le traitement et/ou la prévention des escarres chez les patients présentant des contre-indications à un régime riche en protéines en raison d'une insuffisance rénale, en supplémentant les patients en acides aminés,
dans lequel la composition comprend
comme seule source d'acides aminés: l'alanine, l'histidine, la thréonine et le tryptophane comme substances actives,
de préférence dans lequel les ingrédients actifs alanine et histidine se présentent sous la forme de L-carnosine; et
dans lequel ladite composition se présente sous la forme d'une composition à libération modifiée avec une vitesse de libération ralentie, contrôlée et répétable des substances actives dans le système digestif, et dans lequel la vitesse de libération ralentie, contrôlée et répétable des substances actives est assurée par au moins un additif supplémentaire qui est un agent ralentissant la libération de la substance active dans le système digestif, ou un mélange ou une combinaison de tels additifs.

2. Composition orale nutritionnelle à utiliser selon la revendication 1, dans lequel le rapport pondéral entre la L-carnosine, la thréonine et le tryptophane est de 6:2:1.

3. Composition orale nutritionnelle à utiliser selon les revendications 1 à 2, dans lequel la quantité des ingrédients actifs varie entre 50 % et 80 % du poids total de la composition.

4. Composition orale nutritionnelle à utiliser selon les revendications 1 à 3, dans lequel l'agent ralentissant la libération de l'ingrédient actif dans le système digestif est une substance d'enrobage et/ou une substance formant un support retardateur de libération.

5. Composition orale nutritionnelle à utiliser selon les revendications 1 à 4, dans lequel l'agent ralentissant la libération de l'ingrédient actif dans le système digestif est un système matriciel retardant la libération.

6. Composition orale nutritionnelle à utiliser selon les revendications 1 à 5, dans lequel elle se présente sous la forme d'un comprimé, dragée, capsule dure ou molle, comprimé soluble, comprimé effervescent, comprimé enrobé, comprimé enrobé de sucre, capsule, granulé, poudre à reconstituer en solution, suspension buvable, microcapsule, comprimé/capsule à deux phases comprenant une partie à libération rapide et une partie à libération lente de l'ingrédient actif, comprimé/capsule à libération retardée, comprimé à croquer ou dragée.

7. Composition orale nutritionnelle à utiliser selon les revendications 1 à 6, dans lequel l'agent ralentissant la libération de l'ingrédient actif dans le système digestif est choisi parmi un composé polymère de cellulose, l'éthylcellulose, l'acétate de cellulose, l'hydroxyéthylcellulose, l'hydroxypropylméthylcellulose, la méthylcellulose, la carboxyméthylcellulose sodique, la povidone, la carmellose sodique, l'hypromellose, les gommes naturelles et modifiées, l'agaragar, la gomme carbamate; les alginates, l'acide alginique, les polymères d'acide acrylique, les mucilages; la mélasse, les polysaccharides de mannose, les polysaccharides de galactose, le chitosane, la gélatine, l'amidon, les polyacrylates; les cires, les lipides, les mono-, di- et triglycérides d'acide stéarique et palmitique, l'acide stéarique, le polystyrène, le chlorure de polyvinyle; le sulfate de calcium, le di- et triphosphate de calcium anhydre, l'oxyde de titane, la silice colloïdale; le chlorure de polyvinyle, le polyéthylène, le polystyrène, les polyamides, les résines de silicone, l'Eudragit ou leurs mélanges.

8. Composition orale nutritionnelle à utiliser selon la revendication 7, **caractérisée en ce qu'**elle contient au moins la combinaison d'un composé polymère de cellulose et de povidone; de préférence elle contient au moins la combinaison d'hydroxypropylméthylcellulose et de povidone.
